# EUROPEAN PATENT SPECIFICATION

(11) **EP 4 294 783 B1**
(45) Date of publication and mention of the grant of the patent: **15.10.2025**
(21) Application number: 22718233.4
(22) Date of filing: 12.04.2022
(51) Int. Cl.: C07C 45/62, C07C 49/217, C07C 49/203, C07C 49/21, C07C 49/223, C07C 47/232

(54) **HYDROGENATION OF DIENALS OR DIENONES WITH RHODIUM COMPLEXES UNDER CARBON MONOXIDE FREE ATMOSPHERE**
HYDRIERUNG VON DIENALEN ODER DIENONEN MIT RHODIUMKOMPLEXEN IN KOHLENSTOFFMONOXIDFREIER ATMOSPHÄRE
HYDROGÉNATION DE DIÉNALS OU DE DIÉNONES AVEC DES COMPLEXES DE RHODIUM SOUS UNE ATMOSPHÈRE EXEMPTE DE MONOXYDE DE CARBONE

(30) Priority: 19.04.2021 EP 21169049
(43) Date of publication of application: 27.12.2023
(73) Proprietor: Firmenich SA, 1242 Satigny (CH)
(72) Inventor: SAUDAN, Lionel, 1242 Satigny (CH); QUINTAINE, Julie, 1242 Satigny (CH)
(74) Representative: dsm-firmenich IP
(86) International application number: PCT/EP2022/059703
(87) International publication number: WO 2022/223363

(56) References cited:
- WO-A1-2012/150053

## Description

### Technical field

The present invention relates to the field of catalytic hydrogenation and, more particularly, to the use of a base-free catalytic system comprising a specific rhodium complex for the reduction of a conjugated dienal or dienone into the corresponding deconjugated enal or deconjugated enone.

### Background

The direct selective α-β hydrogenation, *i.e.* of a specific C=C bond, of a conjugated dienal (α,γ-dienal) or conjugated dienone (α,γ-dienone) is a challenging target. Indeed, the hydrogenation may take place at three different sites (two C=C and one C=O). Moreover, in order to render such process attracting for an industrial purpose, it is preferable to achieve the hydrogenation with an acceptable conversion and with a reasonable turn-over (complex load and reaction time).

The selective α-β reduction of conjugated dienals or conjugated dienones has been rarely reported in the literature. The selective α-β hydrogenation of conjugated dienals has been reported in WO2012150053 wherein the selective hydrogenation is performed in the presence of a catalytic system comprising at least a base and at least one complex in the form of a rhodium complex comprising a C₃₄-C₆₀ bidentate diphosphine ligand (L2) coordinating the rhodium. However, the hydrogenations described in this prior arts are always carried out in a presence of a base which represents a drawback for certain substrates leading, in such conditions, to formation of polymer instead of the desired product. In addition, this prior is silent toward the selective α-β hydrogenation of dienones.

Therefore there is still a need for a base-free hydrogenation process allowing the selective α-β hydrogenation of a α,γ-dienal or α,γ-dienone, and if possible within reaction conditions which are applicable industrially. None of the prior art documents reports the use of the specific catalytic system comprising at least one Rh(I) complex obtainable by reacting a suitable Rh precursor having at least one CO ligand with a C₃₄-C₆₀ bidentate diphosphine ligand (L2) having a natural bite-angle comprised between 85° and 130° for the reduction of conjugated dienals or conjugated dienones.

### Description of the invention

In order to overcome the problems aforementioned, the present invention relates to processes for the reduction by hydrogenation, *i.e.* using molecular H₂, of a C₆-C₂₀ conjugated dienal or conjugated dienone into the corresponding deconjugated enal or deconjugated enone, characterized in that said process is carried out in the presence of a catalytic system comprising at least one complex in the form of a rhodium complex comprising a C₃₄-C₆₀ bidentate diphosphine ligand (L2) coordinating the rhodium and at least one CO ligand.

According to a particular embodiment of the invention, the conjugated dienal or conjugated dienone is of formula 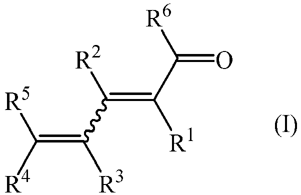 wherein, when taken separately, each of R¹, R², R³, R⁴, R⁵ and R⁶ represents, independently of each other, a hydrogen atom, a phenyl, C₁₋₈ alkyl, C₂₋₈ alkenyl, C₃₋₈ cycloalkyl or C₃₋₈ cycloalkenyl group, each optionally substituted, provided that at least one of said R¹, R², R³, R⁴ and R⁵ is not a hydrogen atom; or R¹ and R² or R² and R³ or R³ and R⁴, when taken together, represent a C₃₋₄ alkanediyl or alkenediyl group optionally substituted; or R¹ and R³ or R² and R⁵, when taken together, represent a C₂₋₃ alkanediyl or alkenediyl group optionally substituted; or R⁴ and R⁵, when taken together, represent a C₄₋₅ alkanediyl or alkenediyl group optionally substituted; or R⁶ and R¹ or R⁶ and R², when taken together, represent a C₂₋₄ alkanediyl or alkenediyl group optionally substituted; or R⁶ and R⁵, when taken together, represent a C₂₋₁₀ alkanediyl or alkenediyl group optionally substituted;
into a deconjugated enal (when R⁶ is a hydrogen atom) or deconjugated enone (when R⁶ is not a hydrogen atom) of formula 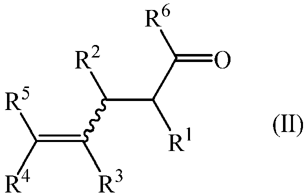 wherein R¹ to R⁶ have the same meaning as defined in formula (I);
said process being carried out in the presence of a catalytic system comprising at least one Rh(I) complex obtainable by reacting a suitable Rh precursor having at least one CO ligand with a C₃₄-C₆₀ bidentate diphosphine ligand (L2) having a natural bite-angle comprised between 85° and 130°.

By the expression "natural bite-angle" it is understood the usual meaning in the art, e.g. as defined in P. W. N. M. van Leeuwen, P. C. J. Kamer, J. N. H. Reek, P. Dierkes, Chem. Rev. 2000, 2741.

Possible substituents of R¹ to R⁶ are one phenyl group, one cyclohexyl, cyclopently, cyclohexenyl or cyclopentenyl group each optionally substituted by one, two or three C₁₋₃ alkyl group, or one, two or three COOR⁷, OCOR⁷, N(R⁷)₂, CN OR⁸ or R⁷ groups, wherein R⁸ is a hydrogen atom or a R⁷ group, wherein R⁷ represents a C₁₋₄ linear or branched alkyl or alkenyl group. Particularly, possible substituents of R¹ to R⁶ are one phenyl group, or one, two or three COOR⁷, OR⁸ or R⁷ groups wherein R⁷ and R⁸ have the same meaning as defined above. According to any one of the embodiments of the invention, only one or two of said R¹ to R⁶ may be optionally substituted, particularly, one or two of said R¹ to R⁵ may be optionally substituted.

The wavy line indicates that the double bond may be in the form of its E or Z isomer or of a mixture thereof; e.g. the C₆-C₂₀ conjugated dienal or conjugated dienone of formula (I) may be in a form of a composition of matter consisting of one or more compounds of formula (I), having the same chemical structure but differing by the configuration of the double bond. In particular, compound (I) comprises two double bond which can be each Z, E or a mixture thereof and compound of formula (II) comprises one double bond which can be Z, E or a mixture thereof. Compound of formula (I) and compound of formula (II) can be in the form of a mixture consisting of isomers E and Z and wherein said isomers E represent at least 50 % of the total mixture, or even at least 75% (i.e a mixture E/Z comprised between 75/25 and 100/0).

For the sake of clarity, by the expression "R¹ and R² ......, when taken together, represent a C₃₋₄ alkanediyl or alkenediyl group" or similar, it is meant the normal meaning understood by a person skilled in the art, i.e. a divalent group formed from alkane or alkene by removal of two hydrogen atoms. In other words, R¹ and R², when taken together, form a C₅₋₆ cycloalkyl or cycloalkenyl group.

It is understood that said compounds (II) can be in a racemic or optically active form, depending on the nature of the substrate and on the complex used.

It is understood that by "alkenyl", "cycloalkenyl" or "alkenediyl" group it is meant here the usual meaning in the art, which is an unsaturated group wherein the unsaturation cannot be conjugated to the carbon-carbon double bonds of the conjugated dienal or conjugated dienone.

The terms "alkyl" and "alkenyl" are understood as comprising branched and linear alkyl and alkenyl groups.

It is understood that by "conjugated dienal", it is meant a compound possessing at least two carbon-carbon double bonds and an aldehyde functional group, the three of them being conjugated, as indicated in formula (I). The term "conjugated dienal" is therefore understood as optionally comprising also compounds having additional non-aromatic carbon-carbon double bonds provided that said additional carbon-carbon double bonds are not conjugated to the ones of the dienal system. It is understood that by "conjugated dienone" or it is meant a compound possessing at least two carbon-carbon double bonds and a ketone functional group, the three of them being conjugated, as indicated in formula (I). The term "conjugated dienone" is therefore understood as optionally comprising also compounds having additional non-aromatic carbon-carbon double bonds provided that said additional carbon-carbon double bonds are not conjugated to the ones of the dienal system.

It is understood that by "deconjugated enal" it is meant a compound possessing at least one γ-δ carbon-carbon double bond and an aldehyde functional group, as indicated in formula (II). The term "deconjugated enal" is therefore understood as optionally comprising also compounds having additional carbon-carbon double bonds provided that said additional non-aromatic carbon-carbon double bonds are not conjugated to the one of the enal system. It is understood that by "deconjugated enone" it is meant a compound possessing at least one γ-δ carbon-carbon double bond and a ketone functional group, as indicated in formula (II). The term "deconjugated enone" is therefore understood as optionally comprising also compounds having additional carbon-carbon double bonds provided that said additional non-aromatic carbon-carbon double bonds are not conjugated to the one of the enone system.

According to any embodiments of the invention, the compounds of formula (I) and (II) is a C₆-C₁₅ compound.

According to any embodiments of the invention, when taken separately, each of R¹, R², R³, R⁴, R⁵ and R⁶ represents, independently of each other, a hydrogen atom, a phenyl, C₁₋₆ alkyl, C₅₋₆ cycloalkyl or C₅₋₆ cycloalkenyl group, each optionally substituted, provided that at least one of said R¹, R², R³, R⁴ and R⁵ is not a hydrogen atom; R³ and R⁴, when taken together, represent a C₃₋₄ alkanediyl group optionally substituted; R⁴ and R⁵, when taken together, represent a C₄₋₅ alkanediyl group optionally substituted. Particularly, when taken separately, each of R¹, R², R³, R⁴, R⁵ and R⁶ represents, independently of each other, a hydrogen atom, a phenyl, C₁₋₄ alkyl, C₅₋₆ cycloalkyl or C₅₋₆ cycloalkenyl group, each optionally substituted, provided that at least one of said R¹, R², R³, R⁴ and R⁵ is not a hydrogen atom; R³ and R⁴, when taken together, represent a C₃₋₄ alkanediyl group optionally substituted; R⁴ and R⁵, when taken together, represent a C₄₋₅ alkanediyl group optionally substituted.

According to any embodiments of the invention, the compounds of formula (I) and (II) are respectively a conjugated dienone and a deconjugated enone; i.e. R⁶ may be a phenyl, C₁₋₈ alkyl, C₂₋₈ alkenyl, C₃₋₈ cycloalkyl or C₃₋₈ cycloalkenyl group, each optionally.

According to any one of the above embodiments, when taken separately, said R¹ may represent a hydrogen atom or a C₁₋₄ alkyl group. Particularly, R¹ may represent a hydrogen atom or a C₁₋₃ alkyl group. Particularly, said R¹ may represent a hydrogen atom, a methyl or a ethyl group. Even more particularly, said R¹ may represent a hydrogen atom.

According to any one of the above embodiments, when taken separately, said R⁶ may represent a hydrogen atom, a C₁₋₄ alkyl or phenyl group. Particularly, R⁶ may represent a hydrogen atom, a C₁₋₃ alkyl group or a phenyl group. Particularly, said R⁶ may represent a hydrogen atom, a methyl, ethyl or phenyl group. Even more particularly, R⁶ may be a methyl or ethyl group.

According to any one of the above embodiments, when taken separately, said R² may represent a hydrogen atom or a C₁₋₄ alkyl group. Particularly, R² may represent a hydrogen atom or a C₁₋₃ alkyl group. Particularly, said R² may represent a hydrogen atom, a methyl or a ethyl group. Even more particularly, said R² may represent a hydrogen atom.

According to any one of the above embodiments, when taken separately, said R³ may represent a hydrogen atom, a C₁₋₄ alkyl group or a phenyl group optionally substituted. Particularly, R³ may represent a hydrogen atom, a C₁₋₃ alkyl group or a phenyl group. Particularly, said R³ may represent a hydrogen atom, a methyl or ethyl group or a phenyl group optionally substituted.

According to any one of the above embodiments, when taken separately, said R⁴ may represent a hydrogen atom, a methyl, ethyl, cyclohexyl, cyclohexenyl cyclopentyl, cyclopentenyl or phenyl group, wherein the cyclohexyl, cyclohexenyl cyclopentyl, cyclopentenyl or phenyl group may be each optionally substituted.

According to any one of the above embodiments, when taken separately, said R⁵ may represent a hydrogen atom, a methyl, ethyl, cyclohexyl, cyclohexenyl cyclopentyl, cyclopentenyl or phenyl group, wherein the cyclohexyl, cyclohexenyl cyclopentyl, cyclopentenyl or phenyl group may be each optionally substituted.

According to any one of the above embodiments, when taken together, said R³ and R⁴, when taken together, represent a C₄ alkanediyl group optionally substituted.

According to any one of the above embodiments, when taken together, said R⁴ and R⁵, when taken together, represent a C₅ alkanediyl group optionally substituted.

According to any one of the above embodiments, the substrate of formula (I) may be one wherein R¹, R² represent each a hydrogen atom, R³, R⁴, R⁵ may represent each a hydrogen atom or a methyl, ethyl, cyclohexyl or phenyl group, wherein the cyclohexyl or phenyl group may be each optionally substituted, provided that at least one of said R¹, R², R³, R⁴ and R⁵ is not a hydrogen atom; or R³ and R⁴, when taken together, represent a C₄ alkanediyl group optionally substituted.

According to any one of the above embodiments, the substrate of formula (I) may be one wherein at least one or two of said R¹, R², R³, R⁴ and R⁵ may be a hydrogen atom.

According to any one of the above embodiments, the substrate of formula (I) may be one wherein two or three of said R¹, R², R³, R⁴ and R⁵ may be a hydrogen atom.

According to any one of the above embodiments, the substituents of said R¹ to R⁶ may be one phenyl group or one, two or three OR⁸ or R⁷ groups, in which R⁸ is a hydrogen atom or a R⁷ group, R⁷ representing a C₁₋₄ linear or branched alkyl group. Preferably said substituents may be a OR⁷ or R⁷ group. According to any one of the embodiments of the invention, only one or two of said R¹ to R⁵ may be optionally substituted. Particularly, R⁷ may represent a C₁₋₃ alkyl group. Particularly, said R⁷ may represent a methyl or a ethyl group.

According to a further embodiment of the invention, the substrate may be a conjugated dienal or a conjugated dienone that will provide a deconjugated enal or deconjugated enone that may be useful in the pharmaceutical, agrochemical or perfumery industry as final product or as an intermediate. Particularly preferred substrate may be a conjugated dienal or a conjugated dienone that will provide a deconjugated enal or deconjugated enone which may be useful in the perfumery industry as final product or as an intermediate.

Non-limiting examples of substrates are the following: (3*E*,5*E*)-5-methyl-6-(p-tolyl)hexa-3,5-dien-2-one, (3*E*,5*Z*)-5-phenylhepta-3,5-dien-2-one, (3*E*)-5-methylocta-3,5-dien-2-one, (3*E*)-5-ethylnona-3,5-dien-2-one, (3*E*)-5-propyldeca-3,5-dien-2-one, (3*E*,5*E*)-6-cyclopentyl-5-methylhexa-3,5-dien-2-one, (3*E*,5*E*)-6-cyclohexyl-5-methylhexa-3,5-dien-2-one, (3*E*,5*E*)-5-(cyclohexylmethylene)hept-3-en-2-one, (*E*)-4-(5,5-dimethylcyclohex-1-en-1-yl)but-3-en-2-one, (*E*)-4-(2,6,6-trimethylcyclohex-1-en-1-yl)but-3-en-2-one, (3*E*,5*E*)-6-(cyclohex-3-en-1-yl)-5-methylhexa-3,5-dien-2-one, (3*E*,5*E*)-5-methyl-7-(2,6,6-trimethylcyclohex-1-en-1-yl)hepta-3,5-dien-2-one, (3*E*,5*E*)-5-ethyl-7-((*S*)-2,2,3-trimethylcyclopent-3-en-1-yl)hepta-3,5-dien-2-one, 6-cyclohexylhepta-3,5-dien-2-one, 6,10-dimethylundeca-3,5,9-trien-2-one, (3*E*,5*E*)-6-phenylhexa-3,5-dien-2-one, (2*E*,4*E*)-4-methyl-5-(*p*-tolyl)penta-2,4-dienal, (2*E*,4*E*)-5-phenylpenta-2,4-dienal, (2*E*,4*E*)-5-phenylhexa-2,4-dienal, (2*E*,4*E*)-4-methyl-5-phenylpenta-2,4-dienal, (2*E,*4*E*)*-2-*methyl-5-phenylpenta-2,4-dienal, (2*E*,4*Z*)-4-phenylhexa-2,4-dienal, (*E*)-3-(4-(tert-butyl)cyclohex-1-en-1-yl)acrylaldehyde, (2*E*,4*E*)-5-cyclohexyl-4-methylpenta-2,4-dienal, (2*E*,4*E*)-5,9-dimethyldeca-2,4,8-trienal, 5,9-dimethyldeca-2,4-dienal, (2*E*,4*E*)-5-cyclopentyl-4-methylpenta-2,4-dienal, 5-methyl-7-phenylhepta-2,4-dienal, (*E*)-3-(5,5-dimethylcyclohex-1-en-1-yl)acrylaldehyde or (2*E*)-4-methyldodeca-2,4-dienal.

According to a particular aspect of any one of the invention's embodiments, the invention's process is also characterized by providing compound (II) with a selectivity above 40%, particularly above 60%, particularly above 80%, particularly above 90%, more particularly above 95%.

According to a particular aspect of any one of the invention's embodiments, the invention's process is also characterized by providing compound (II) with a conversion of the staring compound of above 60%, particularly above 70%, particularly above 80%, particularly above 90%, more particularly above 95%.

Wherein by "deconjugated enal" it is meant the compound (II), by "aldehyde" it is meant the compound (I) wherein both carbon-carbon double bonds have been reduced and by "alcohol" it is meant the aldehyde wherein the carbonyl has also been reduced. Wherein by "deconjugated enone" it is meant the compound (II), by "ketone" it is meant the compound (I) wherein both carbon-carbon double bonds have been reduced.

The hydrogenation reaction can be carried out in the presence or absence of a solvent. In a particular embodiment of the invention, the process is carried out in the presence of a solvent (in general for practical reasons), and any solvent current in hydrogenation reactions can be used for the purposes of the invention. Non-limiting examples include C₆₋₁₀ aromatic solvents such as toluene or xylene, C₁₋₂ halogenated hydrocarbon such as CH₂Cl₂, C₅₋₈ hydrocarbon solvents such as hexane or cyclohexane, C₄₋₉ ethers such as tetrahydrofuran or MTBE, C₃₋₉ esters such as ethyl or methyl acetate, C₃₋₆ ketones such as acetone, polar solvents such as C₁₋₅ primary or secondary alcohols such as isopropanol or ethanol, or mixtures thereof. The choice of the solvent is a function of the nature of the substrate and of the complex and the person skilled in the art is well able to select the most convenient solvent in each case to optimize the hydrogenation reaction.

In the hydrogenation process of the invention, the reaction can be carried out under an atmosphere of pure H₂ or under a mixture of hydrogen and of at least an inert gas, such as N₂ or Ar. Preferably, the atmosphere of the reaction medium is CO-free, *e.g.* the amount of CO present is below 1 ppm. It is understood that in any case the reaction medium is preferably supplied with at least a steochiometric amount of H₂ relative to the substrate; if less than a steochiometric amount of H₂ then it is achieved only a partial conversion of the substrate. In any case, as non-limiting example, one may cite typical H₂ pressure comprised between 10⁵ Pa and 80 x 10⁵ Pa (1 to 80 bar) or even more if desired. Again, a person skilled in the art is well able to adjust the pressure as a function of the complex load and of the dilution of the substrate in the solvent. As examples, one can cite typical pressures of 3 to 50 x 10⁵ Pa (3 to 50 bar), or even of 5 to 20 x 10⁵ Pa (5 to 20 bar).

The temperature at which the hydrogenation can be carried out is comprised between 20°C and 100°C, preferably in the range of between 25°C and 80°C. Of course, a person skilled in the art is also able to select the preferred temperature as a function of the melting and boiling point of the starting and final products as well as the desired time of reaction or conversion.

According to any embodiment of the invention, the process of the invention is performed in absence of base.

As mentioned above, the present invention requires the use of a particular catalytic system comprising at least one Rh(I) complex obtainable by reacting a suitable Rh(I) precursor having at least one CO ligand with a C₃₄-C₆₀ bidentate diphosphine ligand (L2) having a natural bite-angle comprised between 85° and 130°

According to any one of the above embodiments, the Rh(I) complex is a compound obtainable by reacting together:
- a C₃₄-C₆₀ bidentate diphosphine ligand (L2) having a natural bite-angle comprised between 85° and 130°; and
- a suitable Rh precursor of formula

   Rhₚ(CO)_{q}(Z)ᵣ (1) or RhH(CO)(P)₃ (1')

   wherein p is an integer between 1 and 4; q is an integer between 2 and 20; r is an integer between 0 and 1; Z is a coordinated anion and P is a C₃-C₃₀ mono-phosphine.

According to any one of the above embodiments, Z may be a coordinated anion provided that Z is not an halide. Particularly, Z may be selected from the group consisting of acetylacetonate, 1,1,1,5,5,5-hexafluoropentane-2,4-dionate, 2,2,6,6-tetramethylheptane-3,5-dionate, a carboxylate such benzoate, acetate, formiate, pivalate or propionate, an alkoxide such as methoxide, ethoxide, propoxide or butoxide, an allyl such as prop-2-en-1-ide, 3-phenyl-prop-2-en-1-ide, a cyclopentadienyl, a pentamethylcyclopentadienyl and a pentafluorocyclopentadienyl. Particularly, Z may be acetylacetonate.

According to any embodiment of the present invention, P may represent a mono-phosphine of formula PR⁹₃, wherein R⁹ is a C₁-C₁₂ group, such as linear, branched or cyclic alkyl, alkoxy or aryloxy group optionally substituted, substituted or unsubstituted phenyl, diphenyl or naphthyl or di-naphthyl group. More particularly R⁹ may represent a substituted or unsubstituted phenyl, diphenyl or naphthyl or di-naphthyl group. Possible substituents are those cited below for the group R^{b}. Preferably, P is a triphenylphosphine.

According to any embodiment of the present invention, the Rh(0) precursor of formula (1) or (1') may selected from the group consisting of Rh(CO)₂(acac), RhH(CO)(PPh₃)₃, Rh₄(CO)₁₂ and Rh₆(CO)₁₆.

The preparation of the Rh(I) complex is preferably carried out in the presence of a solvent. In a particular embodiment of the invention, said solvent is the same optionally used in the hydrogenation process. However other solvents can be used, and as non-limiting examples one may cite C₆₋₁₀ aromatic solvents such as toluene or xylene, C₅₋₈ hydrocarbon solvents such as hexane or cyclohexane, C₄₋₉ ethers such as tetrahydrofuran or MTBE, polar solvents such as C₁₋₅ primary or secondary alcohols such as isopropanol or ethanol, dichloromethane, water or mixtures thereof. The choice of the solvent is a function of the nature of the substrate and of the complex and the person skilled in the art is well able to select the most convenient solvent in each case to optimize the hydrogenation reaction.

The preparation of the Rh(I) complex can be carried out under an inert, or an essentially carbon monoxide and oxygen free atmosphere, *e.g*. the amount of CO and O₂ present is below 1 ppm. A person skilled in the art knows what is meant by an inert atmosphere. Non-limiting examples of such atmosphere are a nitrogen or argon atmosphere.

In the preparation of the Rh(I) complex, the temperature of the process can be comprised between 0°C and 100°C, preferably in the range of between 10°C and 60°C. Of course, a person skilled in the art is also able to select the preferred temperature as a function of the melting and boiling point of the starting and final products as well as the desired time of reaction or conversion.

According to a particular embodiment, it is believed that the Rh(I) complex can be described as having the formula

[Rh(L2)(CO)(Z)] (2)

wherein L2 and Z has the same meaning as defined above.

According to any one of the above embodiments, L2 can be a compound of formula

(R^{b})₂P-Q-P(R^{b})₂ (A)

wherein each R^{b}, taken separately, represents a C₆₋₁₀ aromatic group optionally substituted or a cyclohexyl group optionally substituted, or the two R^{b} bonded to the same P atom, taken together, represent a 2,2'-oxydiphenyl optionally substituted; and Q represents a C₁₀-C₁₆ metallocenediyl optionally substituted or a group of formula
- a) 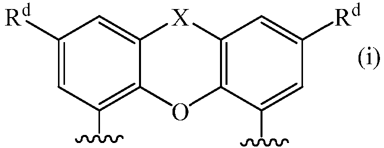 wherein each R^{d} represents a hydrogen atom or a C₁₋₈ alkyl group, and X represents an oxygen or sulfur atom or a C(R¹⁰)₂, Si(R¹¹)₂ or NR¹⁰ group, in which R¹⁰ is a hydrogen atom or a R¹¹ group, R¹¹ representing a C₁₋₄ linear or branched alkyl group, preferably a methyl group; or
- b) 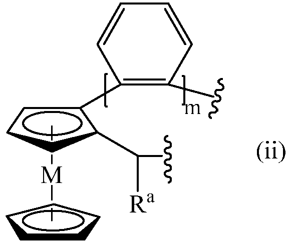 in the form of any one of its enantiomers, and wherein m is 0 or 1, M represents Fe or Ru, and R^{a} represents a hydrogen atom or a C₁₋₄ alkyl group;
and the wavy lines indicate the position of the bond between said Q group and the rest of the compound (A).

According to any one of the above embodiments, Q represents a 1,1'-ferrocenediyl optionally substituted or a group of formula
- a) 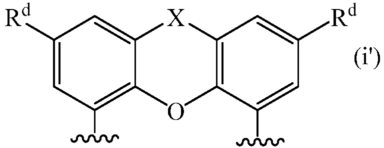 wherein each R^{d} represents a hydrogen atom or a C₁₋₄ alkyl group, and X represents a C(R¹⁰)₂, Si(R¹¹)₂ or NR¹⁰ group, in which R¹⁰ is a hydrogen atom or a R¹¹ group, R¹¹ representing a C₁₋₄ linear or branched alkyl group, preferably a methyl group; or
- b) 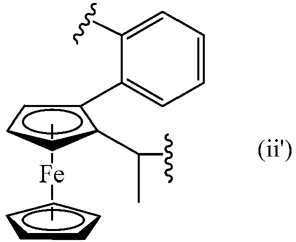 in the form of any one of its enantiomers;
the wavy lines indicate the position of the bond between said Q group and the rest of the compound (A).

According to any one of the above embodiments, in the definition of Q the metallocenediyl is a ferrocenediyl and in particular a 1,1'-diyl group. In formula (ii), in particular, M is Fe.

According to any one of the above embodiments, each R^{b} represents a C₆₋₁₀ aromatic group optionally substituted or a cyclohexyl group optionally substituted.

According to any one of the above embodiments, by "aromatic group or ring" it is meant a phenyl or naphthyl group, and in particular a phenyl group.

According to any one of the above embodiments, each R^{b} represents a phenyl group, a cyclohexyl group, a 3,5-dimethyl-phenyl, a 3,5-di(CF₃)-phenyl, a 3,5-dimethyl-4-methoxy-phenyl group.

According to any one of the above embodiments, the R^{d} represents a hydrogen atom.

According to any one of the above embodiments, X represents a CMe₂, SiMe₂, NH or NMe group.

According to any one of the above embodiments, L2 has a natural bite-angle comprised between 93° and 125°, particularly, comprised between 97° and 125°, particularly comprised between 102° and 125°, particularly comprised between 108° and 125°, even more particularly, comprised between 110° and 125°.

According to any one of the above embodiments, non-limiting examples of possible substituents of R^{b} are one, two, three or four groups selected amongst the halogen atoms, or C₁₋₁₀ alkoxy, alkyl, alkenyl, or perhalo-hydrocarbon groups. The expression "perhalo-hydrocarbon" has here the usual meaning in the art, e.g. a group such as CF₃ for instance. In particular said substituents are one or two halogen atoms, such as F or Cl, or C₁₋₄ alkoxy or alkyl groups, or CF₃ groups.

According to any one of the above embodiments, non-limiting examples of possible substituents of the metallocenediyl or 1,1'-ferrocenediyl group are one or two C₁₋₄ alkyl groups or a CR^{d'}PhN(R^{d"})₂ group, wherein R^{d'} or R^{d"} are a hydrogen atom or a C₁₋₄ alkyl group and Ph is a phenyl group optionally substituted as indicated above for R^{b}. In particular, said substituents are one methyl or one CH(C₆H₅)N(Me)₂ group.

According to any one of the above embodiments, said R^{b}, metallocenediyl or 1,1'-ferrocenediyl groups, one by one or all together, are non-substituted.

According to any one of the above embodiments, the ligand of formula (A) can be in a racemic or optically active form.

As non-limiting examples of L2 ligands, one can cite the following ones: 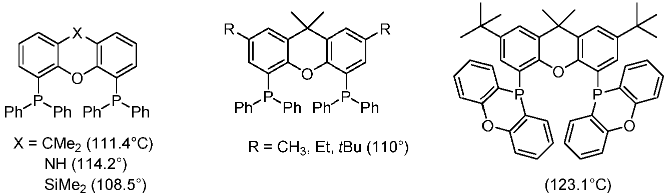 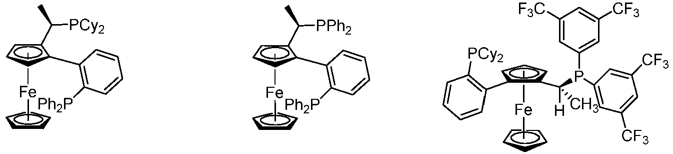 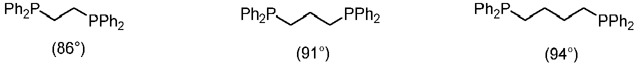 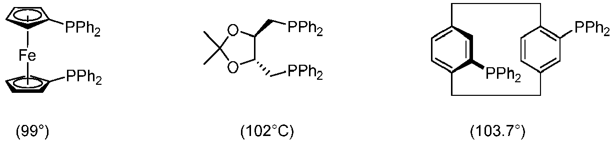 wherein Cy represents a cyclohexyl substituted by one or two C₁₋₄ alkyl groups, Ph represents a phenyl group optionally substituted by one or two C₁₋₄ alkyl groups, one or two trifluoromethyl groups or by one methoxy group;
said compounds being in an optically active form or in a racemic form, if applicable.

The ligands (A) are all known in the prior art and can be obtained by applying standard general methods which are well known in the state of the art and by the person skilled in the art, e.g. see R. P. J. Bronger, P. C. J. Kamer, P. W. N. M. van Leeuwen, Organometallics 2003, 22, 5358 or R. P. J. Bronger, J. P. Bermon, J. Herwig, P. C. J. Kamer, P. W. N. M. van Leeuwen, Adv. Synth. Catal. 2004, 346, 789 or M. Kranenburg, Y. E. M. van der Burgt, P. C. J. Kamer, P. W. N. M. van Leeuwen, K. Goubitz, J. Fraanje Organometallics 1985, 14, 3081 or P. Dierkes, P. W. N. M. van Leeuwen J. Chem. Soc., Dalton Trans. 1999, 1519. Some of said ligands are even commercially available.

According to any one of the above embodiments, L2 ligand is selected from the group consisting of (9,9-dimethyl-9H-xanthene-4,5-diyl)bis(diphenylphosphane) and 4,6-bis(diphenylphosphaneyl)-10H-phenoxazine.

In general, the complexes of formula (2) can be prepared and isolated prior to their use in the process according to the general methods described in the literature. A method is described in the Example.

Moreover, the complexes can be prepared *in situ,* by several methods, in the hydrogenation medium, without isolation or purification, just before their use.

The Rh complex of the invention can be added into the reaction medium of the invention's process in a large range of concentrations. As non-limiting examples, one can cite as complex concentration amounts of complex being greater than 10 ppm, preferably greater than 100 ppm, more preferably greater than 1000 ppm, but less than 50000 ppm, preferably less than 10000 ppm, relative to the amount of substrate. It goes without saying that the optimum concentration of complex will depend, as the person skilled in the art knows, on the nature of the latter, on the nature of the substrate, of the solvent and on the pressure of H₂ used during the process, as well as the desired time of reaction.

### Examples

The invention will now be described in further detail by way of the following examples, wherein the abbreviations have the usual meaning in the art, the temperatures are indicated in degrees centigrade (°C). NMR spectra were acquired using either a Bruker Avance II Ultrashield 400 plus operating at 400 MHz, (¹H) and 100 MHz (¹³C) or a Bruker Avance III 500 operating at 500 MHz (¹H) and 125 MHz (¹³C) or a Bruker Avance III 600 cryoprobe operating at 600 MHz (¹H) and 150 MHz (¹³C). Spectra were internally referenced relative to tetramethyl silane 0.0 ppm. ¹H NMR signal shifts are expressed in δ ppm, coupling constants (*J*) are expressed in Hz with the following multiplicities: s, singlet; d, doublet; t, triplet; q, quartet; m, multiplet; b, broad (indicating unresolved couplings) and were interpreted using Bruker Topspin software. ¹³C NMR data are expressed in chemical shift δ ppm and hybridization from DEPT 90 and DEPT 135 experiments, C, quaternary (s); CH, methine (d); CH₂, methylene (t); CH₃, methyl (q).

### Example 1

### Catalytic hydrogenation of dienone

### A typical experimental procedure is as follows:

In a glove box under argon, a solution of Rh(CO)₂(acac) and Nixantphos **(L2,** structure shown in Table) in dichloromethane was stirred for 30 minutes. A dienone solution in ethanol was charged in a 75 mL autoclave, followed by the solution of [Rh(nixantphos)(CO)(acac)] (0.1 or 0.2 mol%) in dichloromethane. The autoclave was closed, purged with hydrogen gas (10 × 20 bar) and pressurized with hydrogen gas at 20 bar or 50 bar depending on the reaction conditions. The autoclave was placed in an oil bath set at 60°C and the reaction was magnetically stirred for the appropriate time. At the end of reaction, the autoclave was cooled down in an ice bath and depressurized. The reaction mixture was concentrated under reduced pressure. Then the crude product was purified by bulb to bulb distillation to yield the desired ketone, otherwise indicated.

### Hydrogenation of (E)-5-methyl-6-p-tolylhex-5-en-2-one

The autoclave was charged successively with (3E,5E)-5-methyl-6-p-tolylhexa-3,5-dien-2-one (4.081 g, 19.97 mmol) in ethanol (18 mL) and a solution of [Rh(nixantphos)(CO)(acac)] in dichloromethane (2 mL) prepared from Rh(CO)₂(acac) (5.0 mg, 0.02 mmol) and Nixantphos (11.6 mg, 0.02 mmol). The reaction mixture was heated for 2 h 30 min under H₂ pressure (20 bar). Purification by bulb to bulb distillation (bp = 157°C under 1.2 mbar) yielded (E)-5-methyl-6-p-tolylhex-5-en-2-one (3.858 g, 18.69 mmol, 94% yield) as a light yellow oil (GC purity = 98%).
¹H-NMR (400 MHz, CDCl₃): δ=1.84 (d, J = 1.3 Hz, 3H), 2.17 (s, 3H), 2.32 (s, 3H), 2.41-2.44 (m, 2H), 2.62-2.65 (m, 2H), 6.23 (s, 1H), 7.11 ppm (s, 4H).
¹³C-NMR (100 MHz, CDCl₃): δ=208.4 (C), 136.6 (C), 135.7 (C), 135.2 (C), 128.8 (CH), 128.7 (CH), 125.3 (CH), 42.3 (CH2), 34.5 (CH2), 30.0 (CH3), 21.1 (CH3), 17.8 ppm (CH3).

### Example 2

### Catalytic hydrogenation of dienone of (3E,5E)-5-methyl-6-p-tolylhexa-3,5-dien-2-one (S1) with various RhX(Ln) complexes

### General procedure for the screening of various rhodium complexes with diphosphine Ln:

In a glove box, glass vials equipped with a magnetic stirring bar were charged separately with diphosphine **(Ln,** structure shown in Table 1) (1 mol%) and a solution (1 mL) of the rhodium pre-catalyst (1 mol%) in toluene. After stirring at RT for 1 h, a solution (1 mL, 0.5 M, 0.5 mmol) of (3E,5E)-5-methyl-6-p-tolylhexa-3,5-dien-2-one **(S1,** structure shown in Table 7) (0.5 mmol/vial) in toluene was added. The vials were placed in a 75 mL autoclave. The autoclave was closed, purged with hydrogen gas at 20 bar, and finally pressurized with hydrogen gas at 5 bar. The reaction was stirred at room temperature for 20 hours. Then, the autoclave was vented and a sample was taken from each vials and analyzed by GC. The results are shown in Table 2.

**Table 1: List of diphosphines used for the hydrogenation.**

| Diphosphine | Name | Structure |
|---|---|---|
| **L1** | (9,9-dimethyl-9H-xanthene-4,5-diyl) bis(diphenylphosphane) | 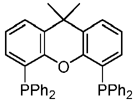 |
| **L2** | 4,6-bis(diphenylphosphaneyl)-10H-phenoxazine | 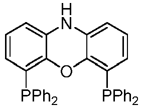 |
| **L3** | (10,10-dimethyl-10H-dibenzo[b,e][1,4] oxasiline-4,6-diyl)bis (diphenylphosphane) | 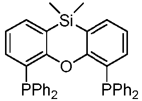 |
| **L4** | 1,1'-diphenylphosphino ferrocene | 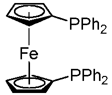 |
| **L5** | (15)-1-[(1R)-1-[Bis[3,5-bis(trifluoro methyl)phenyl]phosphino]ethyl]-2-[2-(dicyclohexylphosphino)phenyl]ferrocene | 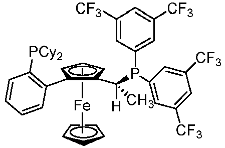 |
| **L6** | 1,2-bis(diphenylphosphaneyl) ethane | 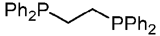 |
| **L7** | 1,3-bis(diphenylphosphaneyl) propane | 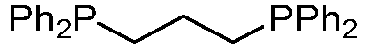 |
| **L8** | 1,4-bis(diphenylphosphaneyl) butane | 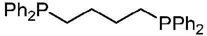 |
| **L9** | (1S)-1-[(1R)-1-[diphenylphosphino]ethyl]-2-[2-(diphenylphosphino)phenyl]ferrocene | 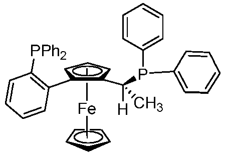 |
| **L10** | (R,R)-Bis(diphenylphosphino-methyl)-2,2-dimethyl-1,3-dioxolane | 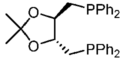 |
| **L11** | (S)-(+)-4,12-Bis(diphenylphosphino)-[2.2]-paracyclophane | 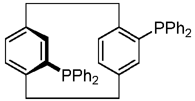 |
| **L12** | (1S)-1-[(1R)-1-[Bis(dicyclohexyl phosphino)]ethyl]-2-[2-(diphenyl phosphino)phenyl]ferrocene | 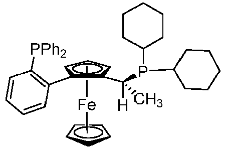 |
| **L13** | (1S)-1-[(1R)-1-[Bis[3,5-bis(trimethyl) phenyl]phosphino]ethyl]-2-[2-(diphenyl phosphino)phenyl]ferrocene | 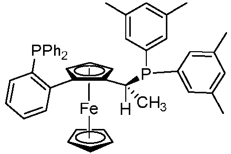 |
| **L14** | (1S)-1-[(1R)-1-[Bis[3,5-bis(trimethyl) phenyl]phosphino]ethyl]-2-[2-(3,5-bis (trimethyl)phenyl) phosphino]ferrocene | 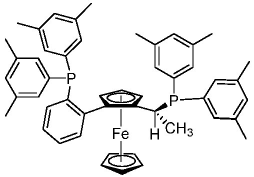 |
| **L15** | (15)-1-[(1R)-1-[Bis[3,5-bis(trifluoro methyl)phenyl]phosphino]ethyl]-2-[2-(diphenylphosphino) phenyl]ferrocene | 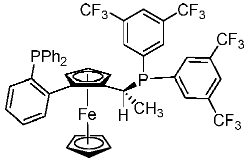 |
| **L16** | (15)-1-[(1R)-1-[Bis[3,5-bis(trifluoro methyl)phenyl]phosphino]ethyl]-2[2-[Bis(4-methoxy-3,5-dimethylphenyl) phosphino]phenyl]ferrocene | 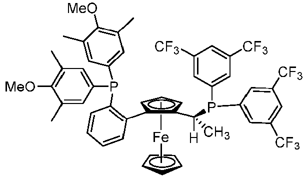 |

**Table 2: Hydrogenation of S1 with various rhodium complexes and L1 under hydrogen (5 bar).**

| N° | Substrate | Com / Base | Rhodium pre-catalyst | Conv. % | Sel. % |
|---|---|---|---|---|---|
| 1* | **S1** | 10000 / 100000 | [Rh(COD)Cl]₂ | 100 | 49 |
| 2** | **S1** | 10000 / 0 | [Rh(COD)₂][BF₄] | 61 | 34 |
| 3 | **S1** | 10000 / 0 | Rh(CO)₂(acac) | 100 | 71 |
| 4*** | **S1** | 10000 / 0 | Rh(CO)₂(acac) | 100 | 88 |

| | | | | | |
|---|---|---|---|---|---|
| Entry 1 and 2 are comparative examples Com/Base = molar ratio in ppm relative to the substrate. Conv. = conversion (in (%), analyzed by GC) of **S1** into the desired product (E)-5-methyl-6-(p-tolyl)hex-5-en-2-one and to any other products, including fully saturated aromatic ketone 5-methyl-6-(p-tolyl)hexan-2-one and deconjugated enone 5-methyl-6-(p-tolyl)hex-4-en-2-one (mixture of E and Z isomers) after the indicated time. Sel. = selectivity (in (%), analyzed by GC) and calculated as 100 x [((E)-5-methyl-6-(p-tolyl)hex-5-en-2-one)]/[((E)-5-methyl-6-(p-tolyl)hex-5-en-2-one) + sum of other products]. * Potassium acetate was added (10 mol%) to the reaction mixture. ** Dichloromethane was used here instead of toluene. *** Reaction stopped after 4 hours. | | | | | |

### Example 3

### Catalytic hydrogenation of dienone of (3E,5E)-5-methyl-6-p-tolylhexa-3,5-dien-2-one (S1) with various Rh(COD)Cl(Ln) complexes - Comparative example (conditions reported in WO2012150053)

### General procedure for the screening of various diphosphines (Ln) with [Rh(COD)Cl]₂ :

In a glove box, glass vials equipped with a magnetic stirring bar were charged separately with the appropriate diphosphines **(Ln)** as shown in Table 1 (1 mol%), [Rh(COD)Cl]₂ (0.5 mol%), potassium acetate (10 mol%) and toluene (1 mL). After stirring at RT for 1 h, a solution (1 mL, 0.5 M, 0.5 mmol) of (3E,5E)-5-methyl-6-p-tolylhexa-3,5-dien-2-one (**S1**, structure shown in Table 7) in toluene was added. The vials were placed in a 75 mL autoclave. The autoclave was closed, purged with hydrogen gas at 20 bar, and finally pressurized with hydrogen gas at 5 bar. The reaction was stirred at room temperature for 20 hours. Then, the autoclave was vented and a sample was taken from each vials and analyzed by GC. The results are shown in Table 3.

**Table 3: Hydrogenation of S1 with various diphosphine (Ln) and [Rh(COD)Cl]₂/AcOK under hydrogen (5 bar).**

| N° | Diphosphine | Com / Base | Conv. % | Sel. % |
|---|---|---|---|---|
| 1 | **L1** | 10000 / 100000 | 100 | 49 |
| 2 | **L4** | 10000 / 100000 | 100 | 0 |
| 3 | **L6** | 10000 / 100000 | 100 | 0 |
| 4 | **L7** | 10000 / 100000 | 100 | 0 |
| 5 | **L8** | 10000 / 100000 | 100 | 0 |

| | | | | |
|---|---|---|---|---|
| Com/Base = molar ratio in ppm relative to the substrate. Conv. = conversion (in (%), analyzed by GC) of **S1** into the desired product (E)-5-methyl-6-(p-tolyl)hex-5-en-2-one and to any other products, including fully saturated aromatic ketone 5-methyl-6-(p-tolyl)hexan-2-one and deconjugated enone 5-methyl-6-(p-tolyl)hex-4-en-2-one (mixture of E and Z isomers) after the indicated time. Sel. = selectivity (in (%), analyzed by GC) and calculated as 100 x [((E)-5-methyl-6-(p-tolyl)hex-5-en-2-one)]/[((E)-5-methyl-6-(p-tolyl)hex-5-en-2-one) + sum of other products]. | | | | |

### Example 4

### Catalytic hydrogenation of dienone of (3E,5E)-5-methyl-6-p-tolylhexa-3,5-dien-2-one (S1) with various Rh(Ln)(CO)(acac) complexes

### General procedure for the screening of rhodium complexes with various diphosphines LI-L4:

In a glove box, glass vials equipped with a magnetic stirring bar were charged separately with the appropriate diphosphines **(L1-L4)** as shown in Table 1 (1 mol%) and a solution (1 mL) of Rh(CO)₂(acac) (1 mol%) in toluene. After stirring at RT for 1 h, a solution (1 mL) of (3E,5E)-5-methyl-6-p-tolylhexa-3,5-dien-2-one (**S1** as shown in Table 7) (0.5 M, 0.5 mmol) in toluene was added. The vials were placed in a 75 mL autoclave. The autoclave was closed, purged with hydrogen gas at 20 bar, and finally pressurized with hydrogen gas at 5 bar. The reaction was stirred at room temperature for 20 hours. Then, the autoclave was vented and a sample was taken from each vials and analyzed by GC. The results are shown in Table 4.

**Table 4: Hydrogenation of S1 with Rh(CO)₂(acac) and various diphosphines (L1-L4) under hydrogen (5 bar).**

| N° | Diphosphine | Com / Base | Conv. % | Sel. % |
|---|---|---|---|---|
| 1 | **L1** | 10000 / 0 | 100 | 71 |
| 2 | **L2** | 10000 / 0 | 58 | 81 |
| 3 | **L3** | 10000 / 0 | 66 | 39 |
| 4 | **L4** | 10000 / 0 | 32 | 47 |

| | | | | |
|---|---|---|---|---|
| Com/Base = molar ratio in ppm relative to the substrate. Conv. = conversion (in (%), analyzed by GC) of **S1** into the desired product (E)-5-methyl-6-(p-tolyl)hex-5-en-2-one and to any other products, including fully saturated aromatic ketone 5-methyl-6-(p-tolyl)hexan-2-one and deconjugated enone 5-methyl-6-(p-tolyl)hex-4-en-2-one (mixture of E and Z isomers) after the indicated time. Sel. = selectivity (in (%), analyzed by GC) and calculated as 100 x [((E)-5-methyl-6-(p-tolyl)hex-5-en-2-one)]/[( (E)-5-methyl-6-(p-tolyl)hex-5-en-2-one) + sum of other products]. | | | | |

### Example 5

### Catalytic hydrogenation of dienone of (3E,5E)-5-methyl-6-p-tolylhexa-3,5-dien-2-one (S1) with various Rh(L1-L2)(CO)(acac) complexes in various solvent

### General procedure for the screening of Rh(L1-L2)(CO)(acac) in various solvent:

In a glove box, a glass vial equipped with a magnetic stirring bar was charged with the appropriate preformed Rh(**L1-L2**)(CO)(acac) complex (0.5 mol%), followed by (3E,5E)-5-methyl-6-p-tolylhexa-3,5-dien-2-one (1mmol, **S1** as shown in Table 7) and the appropriate solvent (2 ml). The vial was placed in a 75 mL autoclave. The autoclave was closed, purged with hydrogen gas at 20 bar, and finally pressurized with hydrogen gas at 5 bar. The reaction was then stirred at 60°C for 4 hours. Then, the autoclave was cooled in an ice/water bath and vented. A sample was taken from the vial and analyzed by GC. The results are shown in Table 5.

**Table 5: Hydrogenation of S1 with Rh(L1-L2)(CO)(acac) in various solvent.**

| N° | Rh**(L1-L2)**(CO)(acac) | solvent | Com / Base | Conv. % | Sel. % |
|---|---|---|---|---|---|
| 1 | **L1** | Toluene | 5000 / 0 | 100 | 89 |
| 2 | **L2** | Toluene | 5000 / 0 | 100 | 93 |
| 3 | **L1** | Acetone | 5000 / 0 | 100 | 93 |
| 4 | **L2** | Acetone | 5000 / 0 | 100 | 92 |
| 5 | **L1** | THF | 5000 / 0 | 100 | 83 |
| 6 | **L2** | THF | 5000 / 0 | 100 | 91 |
| 7 | **L1** | AcOEt | 5000 / 0 | 100 | 87 |
| 8 | **L2** | AcOEt | 5000 / 0 | 100 | 83 |
| 9 | **L1** | EtOH | 5000 / 0 | 100 | 93 |
| 10 | **L2** | EtOH | 5000 / 0 | 100 | 98 |

| | | | | | |
|---|---|---|---|---|---|
| Com/Base = molar ratio in ppm relative to the substrate. Conv. = conversion (in (%), analyzed by GC) of **S1** into the desired product (E)-5-methyl-6-(p-tolyl)hex-5-en-2-one and to any other products, including fully saturated aromatic ketone 5-methyl-6-(p-tolyl)hexan-2-one and deconjugated enone 5-methyl-6-(p-tolyl)hex-4-en-2-one (mixture of *E* and Z isomers) after the indicated time. Sel. = selectivity (in (%), analyzed by GC) and calculated as 100 x [((E)-5-methyl-6-(p-tolyl)hex-5-en-2-one)]/[( (E)-5-methyl-6-(p-tolyl)hex-5-en-2-one) + sum of other products]. | | | | | |

### Example 6

### Catalytic hydrogenation of dienone of (3E,5E)-5-methyl-6-p-tolylhexa-3,5-dien-2-one (S1) with Rh(L2)(CO)(acac) complex at various pressure and temperature

### General procedure for the screening of Rh(L2)(CO)(acac) at various pressure and temperature:

In a glove box, a stainless steel autoclave equipped with a magnetic stirring bar was charged with the preformed Rh(**L2**)(CO)(acac) complex (0.1 mol%), followed by (3E,5E)-5-methyl-6-p-tolylhexa-3,5-dien-2-one (5 mmol, **S1** as shown in Table 7) and EtOH (10 ml). The autoclave was closed, purged with hydrogen gas at 20 bar, and finally pressurized with hydrogen gas at the indicated pressure and the reaction was stirred at the desired temperature for the indicated period of time. Then, the autoclave was cooled in an ice/water bath and vented. A sample was taken from the vial and analyzed by GC. The results are shown in Table 6.

**Table 6: Hydrogenation of S1 with Rh(L2)(CO)(acac) in EtOH at various H₂ pressure and temperature.**

| N° | Com / Base | H₂ (bar) | Temperature (°C) | Time (h) | Conv. % | Sel. % |
|---|---|---|---|---|---|---|
| 1 | 1000 / 0 | 5 | 60 | 4 | 42 | 95 |
| 2 | 1000 / 0 | 20 | 60 | 2.5 | 100 | 98 |
| 3 | 1000 / 0 | 50 | 25 | 1 | 38 | 97 |

| | | | | | | |
|---|---|---|---|---|---|---|
| Com/Base = molar ratio in ppm relative to the substrate. Conv. = conversion (in (%), analyzed by GC) of **S1** into the desired product (E)-5-methyl-6-(p-tolyl)hex-5-en-2-one and to any other products, including fully saturated aromatic ketone 5-methyl-6-(p-tolyl)hexan-2-one and deconjugated enone 5-methyl-6-(p-tolyl)hex-4-en-2-one (mixture of *E* and Z isomers) after the indicated time. Sel. = selectivity (in (%), analyzed by GC) and calculated as 100 x [(E)-5-methyl-6-(p-tolyl)hex-5-en-2-one)]/[( (E)-5-methyl-6-(p-tolyl)hex-5-en-2-one e) + sum of other products]. | | | | | | |

### Example 7

### Catalytic hydrogenation of various dienones (S1-S15) with Rh(L2)(CO)(acac) complex:

### General procedure for the hydrogenation of dienones with Rh(L2)(CO)(acac):

In a glove box, a stainless steel autoclave equipped with a magnetic stirring bar was charged with the preformed Rh(**L2**)(CO)(acac) complex (0.1 mol%) in CH₂Cl₂ (2 ml), followed by the appropriate dienone as shown in Table 7 (20 mmoles) and EtOH (18 ml). The autoclave was closed, purged with hydrogen gas at 20 bar, and finally pressurized with hydrogen gas at 20 bar and the reaction was stirred at 60°C for the indicated period of time. Then, the autoclave was cooled in an ice/water bath and vented. The product was isolated by evaporation of the solvent under reduced pressure and purification by column chromatography on silica gel. The results are shown in Table 8.

**Table 7: List of dienones hydrogenated.**

| Dienones | Name | Structure |
|---|---|---|
| **S1** | (3E,5E)-5-methyl-6-(p-tolyl)hexa-3,5-dien-2-one | 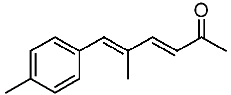 |
| **S2** | (3E,5Z)-5-phenylhepta-3,5-dien-2-one | 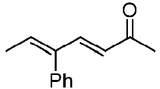 |
| **S3** | (3E)-5-methylocta-3,5-dien-2-one | 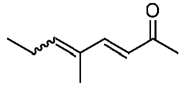 5E, 5Z = 97/3 |
| **S4** | (3E)-5-ethylnona-3,5-dien-2-one | 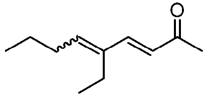 5E, 5Z = 93/7 |
| **S5** | (3E)-5-propyldeca-3,5-dien-2-one | 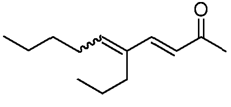 5E, 5Z = 90/10 |
| **S6** | (3E,5E)-6-cyclopentyl-5-methylhexa-3,5-dien-2-one | 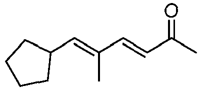 |
| **S7** | (3E,5E)-6-cyclohexyl-5-methylhexa-3,5-dien-2-one | 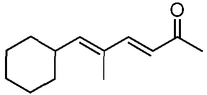 |
| **S8** | (3E,5E)-5-(cyclohexylmethylene)hept-3-en-2-one | 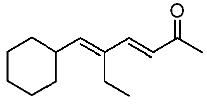 5E, 5Z = 97/3 |
| **S9** | (E)-4-(5,5-dimethylcyclohex-1-en-1-yl)but-3-en-2-one | 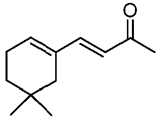 |
| **S10** | (E)-4-(2,6,6-trimethylcyclohex-1-en-1-yl)but-3-en-2-one | 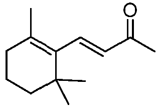 |
| **S11** | (3E,5E)-6-(cyclohex-3-en-1-yl)-5-methylhexa-3,5-dien-2-one | 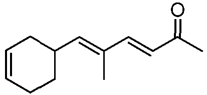 |
| **S12** | (3E,5E)-5-methyl-7-(2,6,6-trimethylcyclohex-1-en-1-yl)hepta-3,5-dien-2-one | 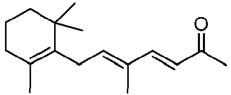 |
| **S13** | (3E,5E)-5-ethyl-7-((S)-2,2,3-trimethylcyclopent-3-en-1-yl)hepta-3,5-dien-2-one | 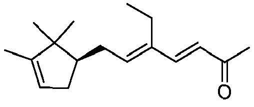 5E, 5Z = 92/8 |
| **S14** | 6-cyclohexylhepta-3,5-dien-2-one | 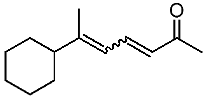 5E, 5Z = 47/53 |
| **S15** | 6,10-dimethylundeca-3,5,9-trien-2-one | 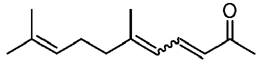 5E, 5Z = 63/37 |
| **S16** | (3E,5E)-6-phenylhexa-3,5-dien-2-one | 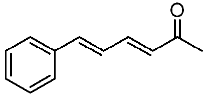 |
| **S17** | (1E,4E)-2-methyl-1,5-diphenylpenta-1,4-dien-3-one | 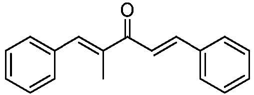 |
| **S18** | (2E,4E)-4-methyl-5-(p-tolyl)penta-2,4-dienal | 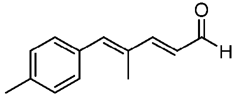 |

**Table 8: Hydrogenation of dienones with Rh(L2)(CO)(acac) in EtOH at 60°C under H₂ pressure (20 bar).**

| N° | Com / Base | Dienones | Time (h) | Conv. % | Sel. % | Yield |
|---|---|---|---|---|---|---|
| 1 | 1000 / 0 | **S1** | 2.5 | 100 | 99 | 94 |
| 2* | 2000 / 0 | **S2** | 2.5 | 100 | >99 | 74 |
| 3 | 1000 / 0 | **S3** | 4 | 100 | >99 | 84 |
| 4 | 1000 / 0 | **S4** | 1.75 | 100 | >99 | 94 |
| 5 | 1000 / 0 | **S5** | 1 | 100 | >99 | 96 |
| 6* | 2000 / 0 | **S6** | 1.5 | 100 | >99 | 97 |
| 7 | 1000 / 0 | **S7** | 4 | 100 | >99 | 94 |
| 8* | 2000 / 0 | **S8** | 1 | 100 | >99 | 98 |
| 9* | 2000 / 0 | **S9** | 2 | 100 | >99 | 97 |
| 10* | 1000 / 0 | **S10** | 2 | 99 | >99 | 98 |
| 11 | 1000 / 0 | **S11** | 3 | 100 | ** | 66 |
| 12* | 2000 / 0 | **S12** | 2.5 | 100 | >99 | 95 |
| 13 | 1000 / 0 | **S13** | 1 | 100 | >99 | 93 |
| 14 | 1000 / 0 | **S14** | 1 | 100 | >99 | 97 |
| 15 | 1000 / 0 | **S15** | 1 | 100 | >99 | 96 |
| 16 | 1000 / 0 | **S16** | 4 | 95 | 86 | ND |
| 17 | 1000 / 0 | **S17** | 3 | 99 | 95 | 98 |

| | | | | | | |
|---|---|---|---|---|---|---|
| Com/Base = molar ratio in ppm relative to the substrate. Conv. = conversion (in (%), analyzed by GC) of **S1-17** into the desired gamma-delta-unsaturated ketone and into any other products, including the fully saturated aromatic ketones after the indicated time. Sel. = selectivity (in (%), analyzed by GC) and calculated as 100 x [desired gamma-delta-unsaturated ketone]/[desired gamma-delta-unsaturated ketone + sum of other products]. Yield = Isolated yield after purification. ND = Not determined. * Reaction performed under 50 bar of hydrogen pressure. ** Hydrogenation of both C=C bonds at the 3,4- and 9,10-positions was observed (15%) along with the desired product (71%) and some unidentified isomers of the fully hydrogenated product (7%). | | | | | | |

### Example 8

### Catalytic hydrogenation of (2E,4E)-4-methyl-5-(p-tolyl)penta-2,4-dienal (S18) with various RhX(L1) complexes:

### General procedure for the screening of various rhodium complexes with diphosphine L1:

In a glove box, glass vials equipped with a magnetic stirring bar were charged separately with Xantphos (**L1**, structure shown in Table 1) (1 mol%), the rhodium pre-catalyst (1 mol%) and CH₂Cl₂ (1 ml). After stirring at RT for 1 h, a solution (1 mL, 1 M, 1 mmol) of (2E,4E)-4-methyl-5-(p-tolyl)penta-2,4-dienal **(S18)** (structure shown in Table 7) in CH₂Cl₂ was added. The vials were placed in a 75 mL autoclave. The autoclave was closed, purged with hydrogen gas at 20 bar, and finally pressurized with hydrogen gas at 50 bar. The reaction was stirred at room temperature for 1 hour. Then, the autoclave was vented and a sample was taken from each vials and analyzed by GC. The results are shown in Table 9.

**Table 9: Hydrogenation of S18 with various rhodium complexes and L1 under hydrogen pressure (50 bar).**

| N° | Substrate | Com / Base | Rhodium pre-catalyst | Conv. % | Sel. % |
|---|---|---|---|---|---|
| 1 | **S18** | 10000 / 0 | [Rh(COD)Cl]₂ | 31 | 88 |
| 2 | **S18** | 10000 / 0 | [Rh(COD)₂][BF₄] | 13 | 96 |
| 3 | **S18** | 10000 / 0 | [Rh(COD)₂][TfO] | 21 | 76 |
| 4 | **S18** | 10000 / 0 | Rh(COD)(acac) | 100 | 69 |
| 5 | **S18** | 10000 / 0 | Rh(CO)₂(acac) | 100 | 87 |

| | | | | | |
|---|---|---|---|---|---|
| Entry 1 to 4 are comparative examples Com/Base = molar ratio in ppm relative to the substrate. Conv. = conversion (in (%), analyzed by GC) of **S18** into the desired aldehyde (E)-4-methyl-5-(p-tolyl)pent-4-enal and any other products, including the saturated alcohol (E)-4-methyl-5-(p-tolyl)pent-4-en-1-ol after 1 hour in this example. Sel. = selectivity (in (%), analyzed by GC) and calculated as 100 x [(E)-4-methyl-5-(p-tolyl)pent-4-enal]/[(E)-4-methyl-5-(p-tolyl)pent-4-enal + (E)-4-methyl-5-(p-tolyl)pent-4-en-1-ol]. | | | | | |

### Example 9

### Catalytic hydrogenation of (2E,4E)-4-methyl-5-(p-tolyl)penta-2,4-dienal (S18) with various Rh(CO)(acac)(Ln) complexes:

### General procedure for the screening of various diphosphines Ln:

In a glove box, glass vials equipped with a magnetic stirring bar were charged separately with the appropriate diphosphines **(Ln)** (1 mol%, as shown in Table 1), Rh(CO)₂(acac) (1 mol%), and CH₂Cl₂ (1 mL). After stirring at RT for 1 h, a solution (1 mL, 0.5 M, 0.5 mmol) of (2E,4E)-4-methyl-5-(p-tolyl)penta-2,4-dienal **(S18)** (structure shown in Table 7) in CH₂Cl₂ was added. The vials were placed in a 75 mL autoclave. The autoclave was closed, purged with hydrogen gas at 20 bar, and finally pressurized with hydrogen gas at 50 bar. The reaction was stirred at room temperature for 1 hour. Then, the autoclave was vented and a sample was taken from each vials and analyzed by GC. The results are shown in Table 10.

**Table 10: Hydrogenation of S18 with various diphosphine (Ln) and Rh(CO)₂(acac) under hydrogen (50 bar).**

| N° | Substrate | Com / Base | Diphosphines | Conv. % | Sel. % |
|---|---|---|---|---|---|
| 1 | **S18** | 5000 / 0 | **L1** | 100 | 95 |
| 2 | **S18** | 5000 / 0 | **L2** | 100 | 87 |
| 3 | **S18** | 5000 / 0 | **L3** | 100 | 83 |
| 4 | **S18** | 10000 / 0 | **L4** | 100 | 98 |
| 5 | **S18** | 10000 / 0 | **L8** | 99 | 97 |
| 6 | **S18** | 10000 / 0 | **L9** | 100 | 86 |
| 7 | **S18** | 10000 / 0 | **L10** | 100 | 97 |
| 8 | **S18** | 10000 / 0 | **L11** | 96 | 99 |
| 9 | **S18** | 5000 / 0 | **L12** | 100 | 61 |
| 10 | **S18** | 5000 / 0 | **L13** | 100 | 98 |
| 11 | **S18** | 5000 / 0 | **L14** | 100 | 97 |
| 12 | **S18** | 5000 / 0 | **L15** | 99 | 91 |
| 13 | **S18** | 5000 / 0 | **L16** | 98 | 97 |

| | | | | | |
|---|---|---|---|---|---|
| Com/Base = molar ratio in ppm relative to the substrate. Conv. = conversion (in (%), analyzed by GC) of **S18** into the desired aldehyde (E)-4-methyl-5-(p-tolyl)pent-4-enal and any other products, including the saturated alcohol (E)-4-methyl-5-(p-tolyl)pent-4-en-1-ol after 1 hour in this example. Sel. = selectivity (in (%), analyzed by GC) and calculated as 100 x [(E)-4-methyl-5-(p-tolyl)pent-4-enal]/[(E)-4-methyl-5-(p-tolyl)pent-4-enal + (E)-4-methyl-5-(p-tolyl)pent-4-en-1-ol]. | | | | | |

### Example 10

### Catalytic hydrogenation of (2E,4E)-4-methyl-5-(p-tolyl)penta-2,4-dienal (S18) with Rh(CO)(acac)(L1) complexes in various solvents:

### General procedure for the screening of Rh(L1)(CO)(acac) in various solvent:

In a glove box, a glass vial equipped with a magnetic stirring bar was charged with the preformed Rh(**L1**)(CO)(acac) complex (0.1 mol%), followed by (2E,4E)-4-methyl-5-(p-tolyl)penta-2,4-dienal **(S18)** (5mmol, as shown in Table 7) and the appropriate solvent (5 ml). The vial was placed in a 75 mL autoclave. The autoclave was closed, purged with hydrogen gas at 20 bar, and finally pressurized with hydrogen gas at 50 bar. The reaction was then stirred at RT for 1 hour. Then, the autoclave was vented. A sample was taken from the vial and analyzed by GC. The results are shown in Table 11.

**Table 11: Hydrogenation of S18 with Rh(L1)(CO)(acac) in various solvent.**

| N° | Solvent | Com / Base | Conv. % | Sel. % |
|---|---|---|---|---|
| 1 | CH₂Cl₂ | 1000 / 0 | 99 | 99 |
| 2 | iPrOH | 1000 / 0 | 99 | 98 |
| 3 | AcOEt | 1000 / 0 | 99 | 99 |
| 4 | THF | 1000 / 0 | 93 | 99 |
| 5 | MTBE | 1000 / 0 | 96 | 99 |
| 6 | acetone | 1000 / 0 | 97 | 99 |
| 7 | toluene | 1000 / 0 | 100 | 97 |

| | | | | |
|---|---|---|---|---|
| Com/Base = molar ratio in ppm relative to the substrate. Conv. = conversion (in (%), analyzed by GC) of **S18** into the desired aldehyde (E)-4-methyl-5-(p-tolyl)pent-4-enal and any other products, including the saturated alcohol (E)-4-methyl-5-(p-tolyl)pent-4-en-1-ol after 1 hour in this example. Sel. = selectivity (in (%), analyzed by GC) and calculated as 100 x [(E)-4-methyl-5-(p-tolyl)pent-4-enal]/[(E)-4-methyl-5-(p-tolyl)pent-4-enal + (E)-4-methyl-5-(p-tolyl)pent-4-en-1-ol]. | | | | |

### Example 11

### Catalytic hydrogenation of (3E,5E)-5-methyl-6-p-tolylhexa-3,5-dien-2-one (S1) with Rh(COD)(acac) or Rh(CO)₂(acac) complexes - Comparative example:

Following the general procedure reported in example 1 and using L2. The results are shown in Table 12.

**Table 12: Hydrogenation of S1 with Rh(COD)(acac) or Rh(CO)₂(acac).**

| Entry | Rh cat. | time (h) | Conv. % | Sel. % |
|---|---|---|---|---|
| 1 | Rh(CO)₂(acac) | 2 h 30 min | >99 | 94 |
| 2 | Rh(COD)(acac) | 2 h 30 min | 19 | 26 |
| 3 | Rh(COD)(acac) | 22 h | 99 | 24 |

| | | | | |
|---|---|---|---|---|
| Entry 2 and 3 are comparative examples Conv. = conversion (in (%), analyzed by GC) of **S1** into the desired product (E)-5-methyl-6-(p-tolyl)hex-5-en-2-one and to any other products, including fully saturated aromatic ketone 5-methyl-6-(p-tolyl)hexan-2-one and deconjugated enone 5-methyl-6-(p-tolyl)hex-4-en-2-one (mixture of E and Z isomers) after the indicated time. Sel. = selectivity (in (%), analyzed by GC) and calculated as 100 x [((E)-5-methyl-6-(p-tolyl)hex-5-en-2-one)]/[( (E)-5-methyl-6-(p-tolyl)hex-5-en-2-one) + sum of other products]. | | | | |

## Claims

1. A process for the reduction by hydrogenation, using molecular H₂, of a C₆-C₂₀ conjugated dienal or conjugated dienone of formula 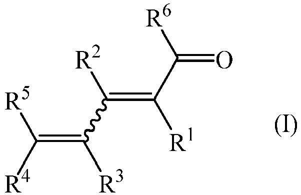
wherein, when taken separately, each of R¹, R², R³, R⁴, R⁵ and R⁶ represents, independently of each other, a hydrogen atom, a phenyl, C₁₋₈ alkyl, C₂₋₈ alkenyl, C₃₋₈ cycloalkyl or C₃₋₈ cycloalkenyl group, each optionally substituted, provided that at least one of said R¹, R², R³, R⁴ and R⁵ is not a hydrogen atom; or R¹ and R² or R² and R³ or R³ and R⁴, when taken together, represent a C₃₋₄ alkanediyl or alkenediyl group optionally substituted; or R¹ and R³ or R² and R⁵, when taken together, represent a C₂₋₃ alkanediyl or alkenediyl group optionally substituted; or R⁴ and R⁵, when taken together, represent a C₄₋₅ alkanediyl or alkenediyl group optionally substituted; or R⁶ and R¹ or R⁶ and R², when taken together, represent a C₂₋₄ alkanediyl or alkenediyl group optionally substituted; or R⁶ and R⁵, when taken together, represent a C₂₋₁₀ alkanediyl or alkenediyl group optionally substituted;
into a deconjugated enal (when R⁶ is a hydrogen atom) or deconjugated enone (when R⁶ is not a hydrogen atom) of formula 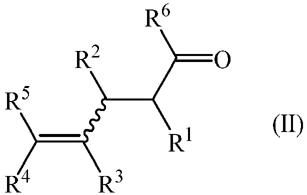
wherein R¹ to R⁶ have the same meaning as defined in formula (I);
said process being carried out in the presence of a catalytic system comprising at least one Rh(I) complex obtainable by reacting a suitable Rh precursor having at least one CO ligand with a C₃₄-C₆₀ bidentate diphosphine ligand (L2) having a natural bite-angle comprised between 85° and 130°.

2. The process according to claim 1, wherein said compound of formula (I) and (II) is a C₆-C₁₅ compound wherein, when taken separately, each of R¹, R², R³, R⁴, R⁵ and R⁶ represents, independently of each other, a hydrogen atom, a phenyl, C₁₋₄ alkyl, C₅₋₆ cycloalkyl or C₅₋₆ cycloalkenyl group, each optionally substituted, provided that at least one of said R¹, R², R³, R⁴ and R⁵ is not a hydrogen atom; R³ and R⁴, when taken together, represent a C₃₋₄ alkanediyl group optionally substituted; R⁴ and R⁵, when taken together, represent a C₄₋₅ alkanediyl group optionally substituted.

3. The process according to any one of claims 1 to 2, wherein said compound of formula (I) and (II) is a compound wherein R¹, R² represent, independently of each other, a hydrogen atom, R³ represents a hydrogen atom, a methyl or ethyl group or a phenyl group optionally substituted; R⁴, R⁵ represent, independently of each other, a hydrogen atom, a methyl, ethyl, cyclohexyl, cyclohexenyl, cyclopentyl, cyclopentenyl or phenyl group, wherein the cyclohexyl, cyclohexenyl cyclopentyl, cyclopentenyl or phenyl group are each optionally substituted, provided that at least one of said R¹, R², R³, R⁴ and R⁵ is not an hydrogen atom; R³ and R⁴, when taken together, represent a C₄ alkanediyl group optionally substituted.

4. The process according to any one of claims 1 to 3, wherein the process of the invention is performed in absence of base.

5. The process according according to any one of claims 1 to 4, wherein the Rh precursor is selected from the group consisting of RhH(CO)(PPh₃)₃, Rh(CO)₂(acac), Rh₄(CO)₁₂ and Rh₆(CO)₁₆.

6. The process according to any one of claims 1 to 5, wherein said Rh(I) complex is a compound of formula
[Rh(L2)(CO)(Z)] (2)
wherein L2 has the same meaning as in claim 1 and Z is a coordinated anion.

7. The process according to any one of claims 1 to 6, wherein L2 is a compound of formula
(R^{b})₂P-Q-P(R^{b})₂ (A)
wherein each R^{b}, taken separately, represents a C₆₋₁₀ aromatic group optionally substituted or a cyclohexyl group optionally substituted, or the two R^{b} bonded to the same P atom, taken together, represent a 2,2'-oxydiphenyl optionally substituted; and Q represents a C₁₀-C₁₆ metallocenediyl optionally substituted or a group of formula
- a) 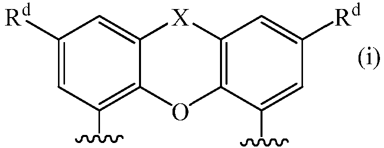 wherein each R^{d} represents a hydrogen atom or a C₁₋₈ alkyl group, and X represents an oxygen or sulfur atom or a C(R¹⁰)₂, Si(R¹¹)₂ or NR¹⁰ group, in which R¹⁰ is a hydrogen atom or a R¹¹ group, R¹¹ representing a C₁₋₄ linear or branched alkyl group, preferably a methyl group; or
- b) 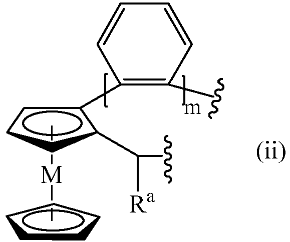 in the form of any one of its enantiomers, and wherein m is 0 or 1, M represents Fe or Ru, and R^{a} represents a hydrogen atom or a C₁₋₄ alkyl group;
and the wavy lines indicate the position of the bond between said Q group and the rest of the compound (A); and
the substituents of R^{b} are one, two, three or four groups selected amongst the halogen atoms, or C₁₋₁₀ alkoxy, alkyl, alkenyl, or perhalo-hydrocarbon groups;
the possible substituents of the metallocenediyl are one or two C₁₋₄ alkyl groups or a CR^{d'}PhN(R^{d"})₂ group, wherein R^{d'} or R^{d"} are a hydrogen atom or a C₁₋₄ alkyl group and Ph is a phenyl group optionally substituted as indicated above for R^{b}.

8. The process according to claim 7, wherein said R^{b} represent each a C₆₋₁₀ aromatic group optionally substituted or a cyclohexyl group optionally substituted.

9. The process according to claim 7, wherein said Q represents a 1,1'-ferrocenediyl optionally substituted or a group of formula
- a) 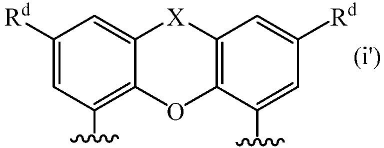 wherein each R^{d} represents a hydrogen atom or a C₁₋₄ alkyl group, and X represents a C(R¹⁰)₂, Si(R¹¹)₂ or NR¹⁰ group, in which R¹⁰ is a hydrogen atom or a R¹¹ group, R¹¹ representing a C₁₋₄ linear or branched alkyl group, preferably a methyl group; or
- b) 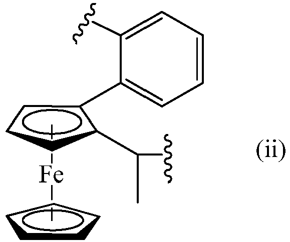 in the form of any one of its enantiomers;
the wavy lines indicate the position of the bond between said Q group and the rest of the compound (A).

10. The process according to claim 7, wherein said L2 has a natural bite-angle comprised between 93° and 125°, preferentially between 97° and 120°.

11. The process according to claim 7, wherein L2 is selected from the group consisting of (9,9-dimethyl-9H-xanthene-4,5-diyl)bis(diphenylphosphane) and 4,6-bis(diphenylphosphaneyl)-10H-phenoxazine.

## Patentansprüche

1. Prozess zur Reduktion, durch Hydrierung unter Verwendung von molekularem H₂, eines konjugierten C₆-C₂₀-Dienals oder konjugierten Dienons der Formel 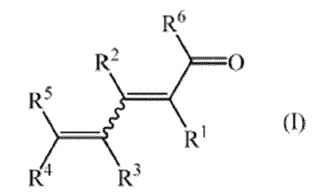
wobei R¹, R², R³, R⁴, R⁵ und R⁶ jeweils unabhängig voneinander ein Wasserstoffatom, eine Phenyl-, C₁₋₈-Alkyl-, C₂₋₈-Alkenyl-, C₃₋₈-Cycloalkyl- oder C₃₋₈-Cycloalkenylgruppe, jeweils gegebenenfalls substituiert, darstellen, vorausgesetzt, dass mindestens eines von R¹, R², R³, R⁴ und R⁵ kein Wasserstoffatom ist; oder R¹ und R² oder R² und R³ oder R³ und R⁴ zusammen eine gegebenenfalls substituierte C₃₋₄-Alkandiyl- oder Alkendiylgruppe darstellen; oder R¹ und R³ oder R² und R⁵ zusammen eine gegebenenfalls substituierte C₂₋₃-Alkandiyl- oder Alkendiylgruppe darstellen; oder R⁴ und R⁵ zusammen eine gegebenenfalls substituierte C₄₋₅-Alkandiyl- oder -Alkendiylgruppe darstellen; oder R⁶ und R¹ oder R⁶ und R² zusammen eine gegebenenfalls substituierte C₂₋₄-Alkandiyl- oder - Alkendiylgruppe darstellen; oder R⁶ und R⁵zusammen eine gegebenenfalls substituierte C₂₋₁₀-Alkandiyl- oder -Alkendiylgruppe darstellen;
in ein dekonjugiertes Enal (wenn R⁶ Wasserstoffatom ist) oder dekonjugiertes Enon (wenn R⁶ kein Wasserstoffatom ist) der Formel 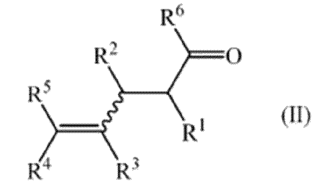
wobei R¹ bis R⁶ die gleiche Bedeutung aufweisen, wie in Formel (I) definiert;
wobei der Prozess in Gegenwart eines katalytischen Systems durchgeführt wird, das mindestens einen Rh(I)-Komplex umfasst, der durch Umsetzen eines geeigneten Rh-Vorläufers erhältlich ist, der mindestens einen CO-Liganden mit einem C₃₄-C₆₀-Bidentat-Diphosphinliganden (L2) aufweist, der einen natürlichen Bisswinkel zwischen 85° und 130° aufweist.

2. Prozess nach Anspruch 1, wobei die Verbindung von Formel (I) und (II) eine C₆-C₁₅-Verbindung ist, wobei, für sich genommen, jedes von R¹, R², R³, R⁴, R⁵ und R⁶ jeweils unabhängig voneinander ein Wasserstoffatom, eine Phenyl-, C₁₋₄ -Alkyl-, C₅₋₆ -Cycloalkyl- oder C₅₋₆ -Cycloalkenylgruppe, jeweils gegebenenfalls substituiert, darstellen, vorausgesetzt, dass mindestens eines von R¹, R², R³, R⁴ und R⁵ kein Wasserstoffatom ist; R³ und R⁴zusammen eine gegebenenfalls substituierte C₃₋₄-Alkandiylgruppe darstellen; R⁴ und R⁵ zusammen eine gegebenenfalls substituierte C₄₋₅-Alkandiylgruppe darstellen.

3. Prozess nach einem der Ansprüche 1 bis 2, wobei die Verbindung der Formel (I) und (II) eine Verbindung ist, wobei R¹, R² unabhängig voneinander ein Wasserstoffatom darstellen, R³ ein Wasserstoffatom, eine Methyl- oder Ethylgruppe oder eine gegebenenfalls substituierte Phenylgruppe darstellt; R⁴, R⁵ unabhängig voneinander ein Wasserstoffatom, eine Methyl-, Ethyl-, Cyclohexyl-, Cyclohexenyl-, Cyclopentyl-, Cyclopentenyl- oder Phenylgruppe darstellen, wobei die Cyclohexyl-, Cyclohexenyl-, Cyclopentyl-, Cyclopentenyl- oder Phenylgruppe jeweils gegebenenfalls substituiert ist, vorausgesetzt, dass mindestens eines von R¹, R², R³, R⁴ und R⁵ kein Wasserstoffatom ist; R³ und R⁴zusammen eine gegebenenfalls substituierte C₄ -Alkandiylgruppe darstellen.

4. Prozess nach einem der Ansprüche 1 bis 3, wobei der erfindungsgemäße Prozess in Abwesenheit einer Base durchgeführt wird.

5. Prozess nach einem der Ansprüche 1 bis 4, wobei der Rh-Vorläufer aus der Gruppe ausgewählt ist, die aus RhH(CO)(PPh₃)₃, Rh(CO)₂(acac), Rh₄(CO)₁₂ und Rh₆(CO)₁₆ besteht.

6. Prozess nach einem der Ansprüche 1 bis 5, wobei der Rh(I)-Komplex eine Verbindung der Formel
[Rh(L2)(CO)(Z)] (2)
ist, wobei L2 dieselbe Bedeutung wie in Anspruch 1 aufweist und Z ein koordiniertes Anion ist.

7. Prozess nach einem der Ansprüche 1 bis 6, wobei L2 eine Verbindung der Formel
(R^{b})₂P-Q-P(R^{b})₂ (A)
ist, wobei jedes R^{b} für sich genommen eine gegebenenfalls substituierte aromatische C₆₋₁₀-Gruppe oder eine gegebenenfalls substituierte Cyclohexylgruppe darstellt, oder die beiden an dasselbe P-Atom gebundenen R^{b} zusammen ein gegebenenfalls substituiertes 2,2'-Oxydiphenyl darstellen; und
Q ein gegebenenfalls substituiertes C₁₀-C₁₆-Metallocendiyl oder eine Gruppe der Formel
- a) 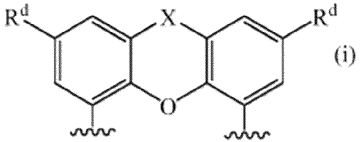 darstellt, wobei jedes R^{d} ein Wasserstoffatom oder eine C₁₋₈-Alkylgruppe darstellt und X ein Sauerstoff- oder Schwefelatom oder eine C(R¹⁰)₂-, Si(R¹¹)₂- oder NR¹⁰-Gruppe darstellt, wobei R¹⁰ ein Wasserstoffatom oder eine R¹¹-Gruppe ist, R¹¹ eine lineare oder verzweigte C₁₋₄-Alkylgruppe, bevorzugt eine Methylgruppe, darstellt; oder
- (b) 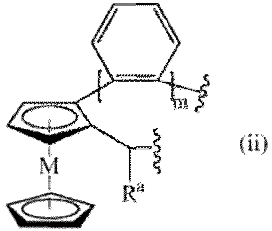 in Form eines seiner Enantiomere, und wobei m 0 oder 1 ist, M Fe oder Ru darstellt und R^{a} ein Wasserstoffatom oder eine C₁₋₄-Alkylgruppe darstellt;
und die Wellenlinien die Position der Bindung zwischen der Q-Gruppe und dem Rest der Verbindung (A) angeben; und
die Substituenten von R^{b} eine, zwei, drei oder vier Gruppen sind, ausgewählt aus den Halogenatomen oder C₁₋₁₀-Alkoxy-, Alkyl-, Alkenyl- oder Perhalogenkohlenwasserstoffgruppen;
die möglichen Substituenten des Metallocendiyls eine oder zwei C₁₋₄-Alkylgruppen oder eine CR^{d'}PhN(R^{d"})₂-Gruppe sind, wobei R^{d'} oder R^{d"} ein Wasserstoffatom oder eine C₁₋₄-Alkylgruppe sind und Ph eine Phenylgruppe ist, die gegebenenfalls substituiert ist, wie oben für R^{b} angegeben.

8. Prozess nach Anspruch 7, wobei jedes R^{b} jeweils eine gegebenenfalls substituierte aromatische C₆₋₁₀-Gruppe oder eine gegebenenfalls substituierte Cyclohexylgruppe darstellt.

9. Prozess nach Anspruch 7, wobei Q ein gegebenenfalls substituiertes 1,1'-Ferrocendiyl oder eine Gruppe der Formel
- a) 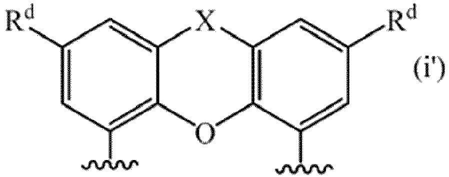 darstellt, wobei jedes R^{d} ein Wasserstoffatom oder eine C₁₋₄-Alkylgruppe darstellt und X eine C(R¹⁰)₂-, Si(R¹¹)₂- oder NR¹⁰-Gruppe darstellt, in der R¹⁰ ein Wasserstoffatom oder eine R¹¹-Gruppe ist, wobei R¹¹ eine lineare oder verzweigte C₁₋₄-Alkylgruppe darstellt, bevorzugt eine Methylgruppe; oder
- (b) 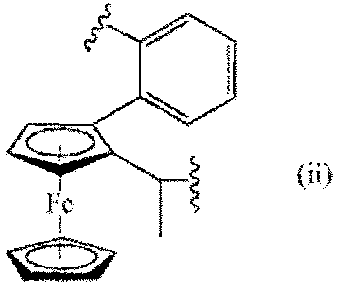
in Form eines seiner Enantiomere;
die Wellenlinien die Position der Bindung zwischen der Q-Gruppe und dem Rest der Verbindung (A) angeben.

10. Prozess nach Anspruch 7, wobei L2 einen natürlichen Bisswinkel zwischen 93° und 125°, bevorzugt zwischen 97° und 120° aufweist.

11. Prozess nach Anspruch 7, wobei L2 aus der Gruppe ausgewählt ist, die aus (9,9-Dimethyl-9H-xanthen-4,5-diyl)bis(diphenylphosphan) und 4,6-Bis(diphenylphosphaneyl)-10H-phenoxazin besteht.

## Revendications

1. Procédé de réduction par hydrogénation, à l'aide de H₂ moléculaire, d'un diénal conjugué en C₆-C₂₀ ou d'une diénone conjuguée de formule 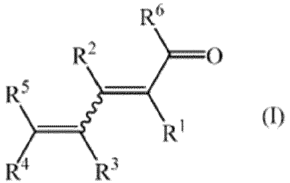
dans laquelle, pris séparément, chacun des R¹, R², R³, R⁴, R⁵ et R⁶ représente, indépendamment les uns des autres, un atome d'hydrogène, un groupe phényle, alkyle en C₁₋₈ , alcényle en C₂₋₈ , cycloalkyle en C₃₋₈ ou cycloalcényle en C₃₋₈ , chacun étant facultativement substitué, à condition qu'au moins l'un desdits R¹, R², R³, R⁴ et R⁵ ne soit pas un atome d'hydrogène ; ou R¹ et R² ou R² et R³ ou R³ et R⁴, pris ensemble, représentent un groupe alcanediyle ou alcènediyle en C₃₋₄ facultativement substitué ; ou R¹ et R³ ou R² et R⁵, pris ensemble, représentent un groupe alcanediyle ou alcènediyle en C₂₋₃ facultativement substitué ; ou R⁴ et R⁵, pris ensemble, représentent un groupe alcanediyle ou alcènediyle en C₄₋₅ facultativement substitué ; ou R⁶ et R¹ ou R⁶ et R², pris ensemble, représentent un groupe alcanediyle ou alcènediyle en C₂₋₄ facultativement substitué ; ou R⁶ et R⁵, pris ensemble, représentent un groupe alcanediyle ou alcènediyle en C₂₋₁₀ facultativement substitué ;
en un énal déconjugué (lorsque R⁶ est un atome d'hydrogène) ou une énone déconjuguée (lorsque R⁶ n'est pas un atome d'hydrogène) de formule 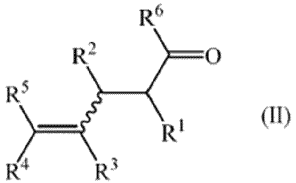
dans laquelle R¹ à R⁶ ont la même signification que celle définie dans la formule (I) ;
ledit procédé étant réalisé en présence d'un système catalytique comprenant au moins un complexe Rh(I) pouvant être obtenu par réaction d'un précurseur de Rh approprié présentant au moins un ligand CO avec un ligand de diphosphine bidentate en C₃₄-C₆₀ (L2) présentant un angle de morsure naturel compris entre 85° et 130°.

2. Procédé selon la revendication 1, dans lequel ledit composé de formule (I) et (II) est un composé en C₆-C₁₅ dans lequel, pris séparément, chacun des R¹, R², R³, R⁴, R⁵ et R⁶ représente, indépendamment les uns des autres, un atome d'hydrogène, un groupe phényle, alkyle en C₁₋₄, cycloalkyle en C₅₋₆ ou cycloalcényle en C₅₋₆, chacun facultativement substitué, à condition qu'au moins l'un desdits R¹, R², R³, R⁴ et R⁵ ne soit pas un atome d'hydrogène ; R³ et R⁴, pris ensemble, représentent un groupe alcanediyle en C₃₋₄ facultativement substitué ; R⁴ et R⁵, pris ensemble, représentent un groupe alcanediyle en C₄₋₅ facultativement substitué.

3. Procédé selon l'une quelconque des revendications 1 à 2, dans lequel ledit composé de formule (I) et (II) est un composé dans lequel R¹, R² représentent, indépendamment l'un de l'autre, un atome d'hydrogène, R³ représente un atome d'hydrogène, un groupe méthyle ou éthyle ou un groupe phényle facultativement substitué ; R⁴, R⁵ représentent, indépendamment l'un de l'autre, un atome d'hydrogène, un groupe méthyle, éthyle, cyclohexyle, cyclohexényle, cyclopentyle, cyclopentényle ou phényle, chaque groupe cyclohexyle, cyclohexényle, cyclopentyle, cyclopentényle ou phényle étant facultativement substitué, à condition qu'au moins l'un desdits R¹, R², R³, R⁴ et R⁵ ne soit pas un atome d'hydrogène ; R³ et R⁴, pris ensemble, représentent un groupe alcanediyle en C₄ facultativement substitué.

4. Procédé selon l'une quelconque des revendications 1 à 3, dans lequel le procédé de l'invention est réalisé en l'absence de base.

5. Procédé selon l'une quelconque des revendications 1 à 4, dans lequel le précurseur de Rh est choisi dans le groupe constitué par RhH(CO)(PPh₃)₃, Rh(CO)₂(acac), Rh₄(CO)₁₂ et Rh₆(CO)₁₆.

6. Procédé selon l'une quelconque des revendications 1 à 5, dans lequel ledit complexe Rh(I) est un composé de formule
[Rh(L2)(CO)(Z)] (2)
dans laquelle L2 a la même signification que dans la revendication 1 et Z est un anion coordonné.

7. Procédé selon l'une quelconque des revendications 1 à 6, dans lequel L2 est un composé de formule
(R^{b})₂P-Q-P(R^{b})₂ (A)
dans laquelle chaque R^{b}, pris séparément, représente un groupe aromatique en C₆₋₁₀ facultativement substitué ou un groupe cyclohexyle facultativement substitué, ou les deux R^{b} liés au même atome P, pris ensemble, représentent un 2,2'-oxydiphényle facultativement substitué ; et
Q représente un métallocènediyle en C₁₀-C₁₆ facultativement substitué ou un groupe de formule
- a) 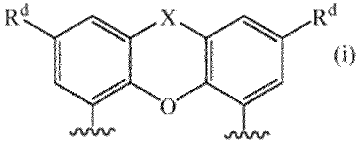 dans laquelle chaque R^{d} représente un atome d'hydrogène ou un groupe alkyle en C₁₋₈, et X représente un atome d'oxygène ou de soufre ou un groupe C(R¹⁰)₂, Si(R¹¹)₂ ou NR¹⁰, dans laquelle R¹⁰ est un atome d'hydrogène ou un groupe R¹¹, R¹¹ représentant un groupe alkyle linéaire ou ramifié en C₁₋₄, de préférence un groupe méthyle ; ou
- (b) 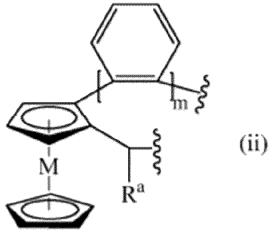 sous la forme de l'un quelconque de ses énantiomères, et dans laquelle m est 0 ou 1, M représente Fe ou Ru, et R^{a} représente un atome d'hydrogène ou un groupe alkyle en C₁₋₄;
et les lignes ondulées indiquent la position de la liaison entre ledit groupe Q et le reste du composé (A) ; et
les substituants de R^{b} sont un, deux, trois ou quatre groupes choisis parmi les atomes d'halogène, ou les groupes alcoxy , alkyle, alcényle ou perhalo-hydrocarbonés en C₁₋₁₀ ;
les substituants possibles du métallocènediyle sont un ou deux groupes alkyle en C₁₋₄ ou un groupe CR^{d'}PhN(R^{d"})₂, dans laquelle R^{d'} ou R^{d"} sont un atome d'hydrogène ou un groupe alkyle en C₁₋₄ et Ph est un groupe phényle facultativement substitué comme indiqué ci-dessus pour R^{b}.

8. Procédé selon la revendication 7, dans lequel lesdits R^{b} représentent chacun un groupe aromatique en C₆₋₁₀ facultativement substitué ou un groupe cyclohexyle facultativement substitué.

9. Procédé selon la revendication 7, dans lequel ledit Q représente un 1,1'-ferrocènediyle facultativement substitué ou un groupe de formule
- a) 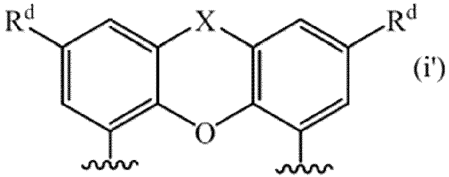 dans laquelle chaque R^{d} représente un atome d'hydrogène ou un groupe alkyle en C₁₋₄, et X représente un groupe C(R¹⁰)₂, Si(R¹¹)₂ ou NR¹⁰, dans laquelle R¹⁰ est un atome d'hydrogène ou un groupe R¹¹, R¹¹ représentant un groupe alkyle linéaire ou ramifié en C₁₋₄, de préférence un groupe méthyle ; ou
- (b) 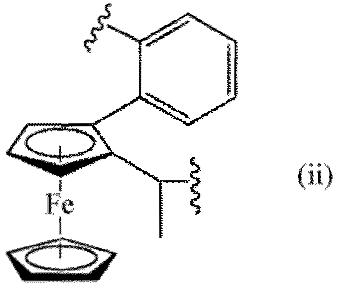
sous la forme de l'un quelconque de ses énantiomères ;
les lignes ondulées indiquent la position de la liaison entre ledit groupe Q et le reste du composé (A).

10. Procédé selon la revendication 7, dans lequel ledit L2 présente un angle de morsure naturel compris entre 93° et 125°, préférentiellement entre 97° et 120°.

11. Procédé selon la revendication 7, dans lequel L2 est choisi dans le groupe constitué par le (9,9-diméthyl-9H-xanthène-4,5-diyl)bis(diphénylphosphane) et la 4,6-bis(diphénylphosphaneyl)-10H-phénoxazine.
